# EUROPEAN PATENT SPECIFICATION

(11) **EP 0 538 335 B1**
(45) Date of publication and mention of the grant of the patent: **11.01.1995**
(21) Application number: 91912826.4
(22) Date of filing: 08.07.1991
(51) Int. Cl.: A61K 9/22, A61K 9/00

(54) **DOSAGE FORM FOR ADMINISTERING A DRUG TO EFFECT CIRCADIAN THERAPY**
DOSIERUNGSFORM ZUR VERABREICHUNG VON ARZNEIMITTELN FÜR DIE ZIRKADIANTHERAPIE
FORME DE DOSAGE POUR L'ADMINISTRATION D'UN MEDICAMENT EN VUE D'UNE THERAPIE CIRCADIENNE

(30) Priority: 12.07.1990 US 551819
(43) Date of publication of application: 28.04.1993
(73) Proprietor: ALZA CORPORATION, Palo Alto California 94303-0802 (US)
(72) Inventor: WONG, Patrick, S.- L., Palo Alto, CA 94306 (US); JAO, Frank, San Jose, CA 95148 (US); THEEUWES, Felix, Los Altos, CA 94022 (US); LAM, Andrew, San Francisco, CA 94122 (US)
(74) Representative: Evans, David Charles
(86) International application number: US9104810
(87) International publication number: WO9200729

(56) References cited:
- EP-A- 0 052 917
- EP-A- 0 238 189
- EP-A- 0 250 083
- DE-A- 3 417 113
- GB-A- 2 189 995

## Description

This invention pertains to a dosage form for administering a drug within a delayed drug-delivery program. More particularly, the invention concerns a dosage form for delivering a drug in a time-varied pattern to a drug recipient.

Presently, pharmacy and medicine provides dosage forms for the constant-rate delivery of a drug to a drug-recipient user. For instance, the prior art provides an infusion pump as disclosed by Perry, Carpenter and Griesenger in U.S. Pat. No. 4,318,400, an oral matrix system as disclosed by Urquhart and Theeuwes in U.S. Pat. No. 4,863,744, an osmotic system as patented by Theeuwes and Higuchi in U.S. Pat. Nos. 3,845,770 and 3,916,899, an osmopolymer-powered system as provided by Wong, Barclay, Deters and Theeuwes in U.S. Pat. No. 4,783,337, an implant as presented by Choi and Heller in U.S. Pat. No. 4,093,709, all designed for the constant-rate delivery of a drug of the longest duration consistent with reproducible therapeutic results.

While the prior art dosage forms provide good therapy and produce their intended results, there are however, some therapeutic programs that require the dose of drug be administered in time-varying patterns of delivery. The time-varying patterns of drug delivery include (a) a drug-free interval followed by a drug pulse of various duration for an extended period of time, (b) an immediate drug dose followed by a drug-free interval followed by a drug-delivery period, (c) a single dose followed by a delayed dose for optimum therapy, and like patterns of drug delivery.

For example, it is known, in Chronobiologia, Vol. 13, pages 239 to 243, (1986), that blood pressure has within-day rhythmicity, and that the highest pressure values are seen often in the morning hours just after waking by the patient. The rise in blood pressure occurring at waking requires a dosage form that is administered on retiring and delivers its drug after a drug-free interval during sleep. This drug delivery pattern provides the need for therapy at the appropriate time, thereby substantially lessening the incidence of a waking elevated blood pressure. Presently a dosage form is unavailable to fulfill this need. It is evident from this discussion, that a critical and unfilled need exists for a dosage form that can deliver a dose of drug in a time-varying pattern of delivery. The need exists for a programmable dosage form that can provide a desired time-profile of drug administration to achieve the intended therapeutic effect.

Dosages forms which provide a drug-free interval are known from GB-A-2 189 995.

In one aspect of the present invention, therefore, there is provided a dosage form for delivering a drug to the gastrointestinal tract of a warm-blooded animal, said dosage form including a compartment which accommodates a first composition including a dosage amount of a drug and a second expandable composition, a semi-permeable wall which surrounds and forms said compartment and exit means in said wall; wherein, in service, fluid from the gastrointestinal tract enters into the compartment by osmosis across the semi-permeable wall thereby forming a solution of said drug and causing or allowing the expandable composition to expand and push against the first composition, thereby to deliver said solution of drug progressively to the gastrointestinal tract via said exit means; characterised in that said first composition further includes delaying means having a greater solubility and a greater osmotic pressure gradient across the semi-permeable wall than the drug such that in service said delaying means is delivered from the dosage form before the drug to provide a substantially drug-free interval.

In another aspect, the invention may be a dosage form characterised in that the delaying means is selected from one or more of sodium chloride, potassium chloride, magnesium sulfate, magnesium chloride, potassium sulfate, sodium sulfate, potassium acid phosphate, mannitol phosphate, sodium acid phosphate, tartaric acid and citric acid.

Accordingly, in the light of the above presentation, it is an immediate object of this invention to provide a novel dosage form that overcomes the shortcomings of the prior art and fully satisfies the critical and unfilled need for the dosage form.

Another object of this invention is to provide a dosage form possessing time-varying patterns of drug delivery for achieving optimum therapy.

Another primary object of this invention is to provide a programmable drug-delivery dosage form that substantially fulfills the pressing need of the prior art and also represents an unexpected improvement in the dispensing art.

Another object of the present invention is to provide a programmable drug-delivery system adapted as a dosage form for a rate-programmed drug delivery at time-varying patterns.

Another object of the present invention is to provide a dosage form comprising structural means for providing drug-free intervals followed by drug-delivery periods of various time durations.

Another object of the present invention is to provide a dosage that can deliver an instant-pulse dose of a therapeutic drug, followed by a delayed delivery of drug, and then deliver a dose of drug.

Another object of the present invention is to provide a dosage form comprising two timed spaced-apart doses of drug in a single dosage form.

Another object of the invention is to provide a dosage form comprising two doses of drug in a single dosage form that can be used for twice a day dosing of drug.

Another object of the present invention is to provide a novel dosage form manufactured in the form of a drug delivery device comprising means for delivering a pulsed dose of drug to a human, means for providing a drug-free interval, and then providing a recurring pulse dose of drug to the human.

Another object of the invention is to make available a dosage form that delivers a first or instant dose of drug at bed-time for providing drug during sleep, and a second or delayed dose of drug early in the morning for providing drug therapy on awakening from sleep.

Another object of the invention is to make available a dosage form that delivers a first dose of drug in the morning and a second dose of drug in the afternoon thereby providing two spaced apart doses of drug therapy from a single dosage form.

Other objects, features, and advantages of the invention will be more apparent to those versed in the dispensing art from the following specification, taken in conjunction with the drawing figures and the accompanying claims.

Following is a description by way of example only and with reference to the accompanying drawings of methods of carrying the present invention into effect.

In the drawing figures, which are not drawn to scale, but are set forth to illustrate various embodiments of the invention, the drawing figures are as follows:
Figure 1 is a view of a dosage form provided by the invention, which dosage form is designed, sized and adapted for admitting into a biological environment of use for time-varying patterns of drug delivery including drug-free intervals between drug doses;
Figure 2 is a view of the dosage form of Figure 1, wherein Figure 2 depicts a dose of drug on the exterior surface of the dosage form for administering a drug instantly in a short period of time to a recipient followed by a drug-free interval before drug is delivered from the interior of the dosage system;
Figure 3 is an opened view of the dosage form of Figure 1 for illustrating the internal structure of the dosage form for proving a time-interval, drug-free period followed by a drug delivery period over time;
Figure 4 is a view of the dosage form for Figure 1, wherein the dosage form of Figure 4 comprises a plurality of passageways for delivering a drug from the dosage form; and,
Figure 5 is a graph that depicts the delay of drug delivery followed by the delivery of a drug from a dosage form provided by the invention.

In the drawing figures and in the specification, like parts in related figures are identified by like numbers. The terms appearing earlier in the specification, and in the description of the drawing figures, as well as embodiments thereof, are further described elsewhere in the disclosure.

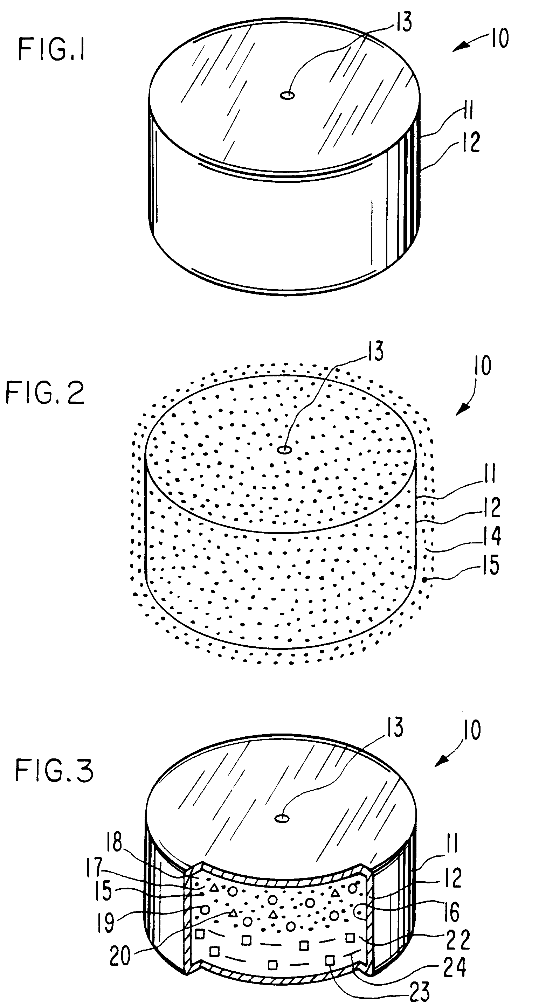

Turning now to the drawing figures in detail, which drawing figures are an example of the dosage forms provided by the invention, and which examples are not to be construed as limiting, one example of the dosage form is illustrated in Figure 1 and it is designed by the numeral 10. In Figure 1, dosage form 10 comprises a body 11 comprising a wall 12 that surrounds and forms an internal compartment, not seen in Figure 1. Dosage form 10 further comprises at least one exit means 13, or more than one exit means 13 on the same or on different surfaces, for connecting the interior of dosage form 10 with exterior of dosage form 10.

Dosage form 10, as seen in opened section in drawing figure 2, depicts an optional embodiment of the invention, that comprises an external coat 14 on the exterior surface of wall 12. Coat 14 is a composition comprising 1 mg to 100 mg of drug 15, represented by dots. Exterior coat 14 provides instant drug 15 according to the programmable delivery patterns provided by dosage form 10. Drug 15 in exterior coat 14 is blended with an aqueous soluble film-forming carrier such as methylcellulose, hydroxypropylcellulose, hydroxypropylmethylcellulose, blends of hydroxypropylcellulose and hydroxypropylmethylcellulose, optionally blended with a plasticizer such as polyethylene glycol or acetylated triglycerides. Coat 14 provides instant drug therapy, as film coat 14 dissolves or undergoes dissolution in the presence of fluid and concurrently therewith releases and delivers drug 15 to a drug receptor. Coat 14 comprising drug 15 provides (1) instant drug followed by a drug-free interval, and (2) it essentially overcomes the time required for drug 15 to be delivered from the interior of dosage form 10. A start-up time is needed for dosage form 10 to imbibe exterior fluid through wall 12 into its interior for dosage form 10 to hydrodynamically and osmotically dispense drug from its interior through exit means 13.

Dosage form 10, as provided by this invention, and as seen in the above drawing figures can be manufactured for administering a drug by the oral route, and in another embodiment, dosage form 10 comprising an exterior drug can be sized and shaped for administering a drug by the sublingual or by the buccal routes. The sublingual and buccal routes can be used for quicker therapy and they can be used when a smaller dose of drug is needed for therapy. The buccal and sublingual routes can be used also as a by-pass of the first pass of hepatic metabolism of a drug. The sublingual or buccal routes can be used for administering the first pulse of drug, followed by permitting dosage form 10 to enter the stomach for subsequent drug delivery.

In drawing figure 3, dosage form 10 is manufactured as an osmotic device, and it is seen in opened view at 16. In drawing figure 3, dosage form 10 comprises body 11, wall 12, that is sectioned at 16, and which wall 12 surrounds and defines an internal compartment 17. Wall 12 comprises at least one exit means 13 that connects compartment 17 with the exterior of dosage form 10. Dosage form 10 can comprise more than one exit means 13, as presented later in the specification.

Wall 12 of dosage form 10, comprises totally, or in at least a part, a composition that is permeable to the passage of an exterior fluid present in the environment of use. Wall 12 is substantially impermeable to the passage of drug and other optional ingredients that may be present in compartment 17. The semipermeable wall 12 is substantially inert, that is, it maintains its physical and chemical integrity during the dispensing of a drug from dosage form 10 and it is nontoxic. Wall 12 in one preferred embodiment is formed totally or in at least a part of a member selected from the group consisting of a cellulose ether polymer, a cellulose ester polymer, or a cellulose ester-ether polymer. The cellulosic polymers have a degree of substitution, D.S., on their anhydroglucose unit, of from greater than 0 up to 3 inclusive. By degree of substitution is meant the average number of hydroxyl groups originally present on the anhydroglucose unit comprising the cellulose polymer that are replaced by a substituting group. Representative materials include a member selected from the group consisting of cellulose acylate, cellulose diacylate, cellulose triacylate, cellulose acetate, cellulose diacetate, cellulose triacetate, mono, di and tricellulose alkanylates, mono, di, and tricellulose aroylates. Exemplary polymers include cellulose acetate having a D.S. up to 1 and an acetyl content up to 21%; cellulose acetate having an acetyl content of 32 to 39.8%; cellulose acetate having a D.S. of 1 to 2 and an acetyl content of 21 to 35%; cellulose acetate having a D.S. of 2 to 3 and an acetyl content of 35 to 44.8%. More specific cellulosic polymers include cellulose propionate having a D.S. of 1.8 and a propyl content of 39.2 to 45% and a hydroxyl content of 2.8 to 5.4%; cellulose acetate butyrate having a D.S. of 1.8, an acetyl content of 13 to 15% and a butyryl content of 34 to 39%; cellulose acetate butyrate having an acetyl content of 2 to 29%, a butyryl content of 17 to 53% and a hydroxyl content of 0.5 to 4.7%; cellulose triacylates having a D.S. of 2.9 to 3 such as cellulose triacetate, cellulose trivalerate, cellulose trilaurate, cellulose tripalmitate, cellulose trisuccinate, and cellulose trioctanoate; cellulose diacylates having a D.S. of 2.2 to 2.6 such as cellulose disuccinate, cellulose dipalmitate, cellulose dioctanoate, cellulose dipentanoate, co-esters of cellulose such as cellulose acetate butyrate and cellulose acetate propionate.

Additional semipermeable wall forming polymers include acetaldehyde dimethyl cellulose acetate, cellulose acetate ethyl carbamate, cellulose acetate methyl carbamate, cellulose acetate dimethyl aminoacetate, semipermeable polyamides; semipermeable polyurethanes; semipermeable sulfonated polystyrenes; semipermeable cross-linked selectively permeable polymers formed by the coprecipitation of a polyanion and a polycation as disclosed in U.S. Pat. Nos. 3,173,876; 3,276,586; 3,541,005; 3,541,006, and 3,546,142; semipermeable polymers as disclosed by Loeb and Sourirajan in U.S. Pat. No. 3,133,132; semipermeable lightly cross-linked polystyrene derivatives; semipermeable cross-linked poly(sodium styrene sulfonate); and semipermeable cross-linked ply(vinylbenzyltrimethyl ammonium chloride). The polymers are known to the art in U.S. Pat. Nos. 3,845,770; 3,916,899; and 4,160,020; and in *Handbook of Common Polymers* by Scott, J.R. and Roff, W.J., 1971, published by CRC Press, Cleveland, Ohio.

In another embodiment, wall 12 of dosage form 10 of drawing figure 3 optionally comprises from 0 weight percent (wt %) to 30 wt % of a member selected from the group consisting of a cellulose ether selected from the group consisting of a hydroxypropylcellulose and a hydroxypropylmethylcellulose, and from 0 wt % to 30 wt % of a polyethylene glycol. The total weight of all components comprising wall 12 is equal to 100 wt %.

Dosage form 10 of drawing figure 3, comprises a first composition, also identified as a first layer 18 positioned in compartment 17 next to passageway 13. First layer 18 comprises a drug 15, identified by dots. The term "drug" as used herein includes any physiologically or pharmacologically active substance that produces a local or systemic effect in animals, including warm blooded mammals; humans and primates; avians; household, sport and farm animals; laboratory animals; fishes; reptiles and zoo animals. The term "physiologically" as used herein denotes the administration of a drug to produce generally normal levels and functions. The term "pharmacologically" denotes generally variations in response to the amount of drug administered to the host. See *Stedman's Medical Dictionary*, 1966, published by Williams and Wilkins, Baltimore, MD.

The beneficial drug 15 that can be delivered includes inorganic and organic compounds without limitation, including drugs that act on the peripheral nerves, adrenergic receptors, cholinergic receptors, nervous system, skeletal muscles, cardiovascular system, smooth muscles, blood circulatory system, synaptic sites, neuroeffector junctional sites, endocrine system, hormone systems, immunological system, reproductive system, skeletal system, autacoid systems, alimentary and excretory systems, inhibitory of autocoid systems, alimentary and excretory systems, inhibitory of autocoids and histamine systems. The active drug that can be delivered for acting on these recipients include anticonvulsants, analgesics, antiparkinson, anti-inflammatories, calcium antagonists, anesthetics, antimicrobials, antimalarials, antiparasites, antihypertensives, antihistamines, antipyretics, alpha-adrenergic agonist, alpha-blockers, biocides, bactericides, bronchial dilators, beta-adrenergic blocking drug, contraceptives, cardiovascular drugs, calcium channel inhibitors, depressants, diagnostics, diuretics, electrolytes, hypnotics, hormonals, hyperglycemics, muscle contractants, muscle relaxants, ophthalmic, psychic energizers, parasympathomimetics, sedatives, sympathomimetics, tranquilizers, urinary tract drugs, vaginal drugs, vitamins, nonsteroidal anti-inflammatory drugs, angiotensin converting enzymes, and polypeptide drugs.

Drug 15, in one preferred embodiment, comprises a calcium channel blocker. The calcium channel blocker generally blocks the influx of calcium ions into smooth and cardiac muscles and thus they possess patent cardiovascular effects. The calcium blockers are useful for alleviating symptoms of chronic heart disease, for the management of angina pectoris and myocardial, and hypertension, while exhibiting a low incidence of side effects. Drug 15, in one presently preferred embodiment, comprises a calcium channel blocker consisting of a member selected from the group consisting of phenylalkylamine calcium blockers, benaothiazepine calcium blockers, diphydropyridines calcium blockers, quinazolines calcium antagonist, quinoxolines calcium antagnist, piperazines calcium antagonist, caroverine, diltiazem, fendiline, gallopamil, isradipine, lidoflazine, nifedipine, nilvadipine, nimodipine, nicardipine, nitrendipine, nisoldipine, and verapamil. Another presently preferred group of drugs that can be administered after a delayed interval includes drugs that are vasodilators. The vasodilators include nitrites, nitrates, their esters, such as their esters of sugars and polyols. The vasodilators generally possesses a member selected from the group consisting of ONO and ONO₂. The drugs include a member selected from the group consisting of amyl nitrite, glyceryl trinitrate, also known as nitroglycerin, nitroglycerin absorbed on lactose, octyl nitrite, sodium nitrite, erythrityl tetranitrate, isosorbide dinitrate, mannitol hexanitrate, pentaerythritol tetranitrate, pentrintrol, triethanolamine trinitrate, and trolnitrate phosphate. The vasodilators are used to relieve the pain associated with angina pectoris, for the prevention of angina, for treating hypertension, for the relaxation of involuntary muscles of blood vessels mainly arteries and arterioles, for increasing the flow of blood therein, and for increasing oxygenation from vasodilation, mainly for increasing the supply of oxygen to the heart. The amount of drug 15 in first composition 18 generally is from 0.05 ng to 2 g or more, with individual dosage forms containing, for example, 25 ng, 1 mg, 5 mg, 10 mg, 25 mg, 50 mg, 100 mg, 150 mg, 200 mg, 250 mg, 500 mg, 750 mg, 1.0 g, 1.2 g, and 1.5 g. The drugs are known to the art in *Pharmaceutical Sciences*, 14th Ed., edited by Remington, (1979) published by Mack Publishing Co., Easton, PA; *The Drug*, *The Nurse*, *The Patient*, *Including Current Drug Handbook*, by Falconer, et al., (1974-1976) published by Saunder Company, Philadelphia, PA; *Medicinal Chemistry*, 3rd Ed., Vol. 1 and 2, by Burger, published by Wiley-Interscience, New York; and in *Physician's Desk Reference*, 38 Ed., (1984) published by Medical Economics Co., Oradell, NJ.

First composition 18 comprises also means 19 for effecting a drug-free interval after dosage form 10 is administered orally to a patient. Means 19, represented by circles, delays the delivery of drug 15 by means 19 preferentially being delivered first from compartment 17 followed by the delivery of drug 15 from dosage form 10. Means 19 exhibits a greater solubility in fluid imbibed through semipermeable wall 12 into dosage form 10 then drug 15, and means 19 exhibits also a greater osmotic pressure gradient across wall 12. These dual kinetic properties lead to means 19 delivered before drug 15 is delivered from dosage form 10. This kinetic activity produces a drug-free interval up to 4 to 4 1/2 hours followed by a drug delivery period up to 20 hours.

For the purpose of this invention, the solubility of means 19 or drug 15 in a fluid can be determined by various art known techniques. One method consists in preparing a saturated solution of means 19 or of drug 15 for example, a fluid plus the means or the drug and ascertaining by analysis the amount of means or drug present in a definite quantity of the fluid. A simple apparatus for this purpose consists of a test tube of medium size fastened upright in a water bath maintained at constant temperature and pressure, for example 37.5°C and one atmosphere. The fluid and means or drug are placed in the tube and stirred by means of a motor driven rotating glass spiral. After a given period of stirring, a definite weight of the fluid is analyzed and the stirring continued for an additional period of time. If the analysis shows no increase after successive periods of stirring, in the presence of excess solid means or drug in the fluid, the solution is saturated and the results are taken as the solubility of the means or drug in the fluid. Numerous other methods are available for the determination of the solubility of the means or the drug in a fluid. Typical methods used for the measurement of solubility are chemical analysis, measurement of density, refractive index, and electrical conductivity. Details of the various methods for determining solubilities are described in United States Public Health Service Bulletin, No. 67 of the Hygienic Laboratory: Encyclopedia of Science and Technology, Vol. 12, pages 542 to 556, 1971, McGraw-Hill, Inc., and "Encyclopaedic Dictionary of Physics, Vol. 6, pages 545 to 557, 1962, Pergamon Press Inc. The osmotic pressure of means 19 or of drug 15 is measured in a commercially available osmometer that measures the vapor pressure difference between pure water and the means or drug solution to be analyzed, and according to standard thermodynamic principles, the vapor pressure ratio is converted into osmotic pressure difference. The osmotic pressure of a saturated solution of means 19 or of drug 15 is measured at 37°C in water, and the osmotic pressure is expressed in ATM. The osmotic pressure is measured using an osmometer. An osmometer used for the present measurements is identified as Model 302B, Vapor Pressure Osmometer, manufactured by the Hewlett Packard Co., Avondale, PA.

In another technique, the osmotic pressure is measured using a porous cell impregnated with copper ferrocyanide filled with water and immersed in a vessel containing the aqueous solution. The pressure is measured by means of an attached manometer. The system is allowed to stand until there is no further increase in pressure. Then the osmotic pressure is just balanced by the hydrostatic pressure in the column of solution. A pressure up to several hundred atmospheres can be measured by using a capillary manometer for the pressure measurement. Other methods that can be used for measuring osmotic pressure include using this apparatus and applying a pressure to the solution sufficient to balance an osmotic pressure read in a pressure gauge. Calculations of pressure can be made also from changes in the refractive index of water on compression, and from the application of piezoelectric gauges. The techniques for measuring osmotic pressure are disclosed in Physical Chemistry by Walter J. Moore, Third Edition, pages 136 to 137, (1962) published by Prentice-Hall, Inc., Englewood Cliffs, NJ.

Representative of means 19 comprises a member selected from the group consisting of sodium chloride, potassium chloride, magnesium sulfate, magnesium chloride, potassium sulfate, sodium sulfate, potassium acid phosphate, mannitol phosphate, sodium acid phosphate, tartaric acid, citric acid, and mixtures thereof. The amount of means 19 in first composition 18 is from 1 mg to 750 mg.

First composition 18 comprises also pharmaceutically acceptable composition forming ingredients, represented by triangles 20, such as a binder, lubricant, disintegrant, and color agent. Typically first composition 18 comprises 5 to 50 mg of a disintegrant such as a member selected from the group consisting of poly(vinyl pyrrolidone), lightly cross-linked poly(vinyl pyrrolidone), corn starch, potato starch, bentonite, and citrus pulp. The first composition 18 comprises optionally from 0.25 mg to 5 mg of a lubricant such as a member selected from the group consisting of stearic acid, zinc palmitate, magnesium stearate, calcium stearate, zinc stearate, aluminum stearate, magnesium oleate, halogenated vegetable oil, pulverized teflon, and pulverized talc.

Dosage form 10 comprises a second composition 22 or a push layer 22. The push layer 22 comprises an osmopolymer 23, identified by squares, suitable for forming push layer 22. The second layer 22 comprises an osmopolymer that exhibits fluid imbibition properties. The osmopolymers are swellable, hydrophilic polymers which osmopolymers interact with water and aqueous biological fluids and swell or expand to an equilibrium state. The osmopolymers exhibit the ability to swell in water and retain a significant portion of the imbibed water within the polymer structure. The osmopolymers swell or expand to a very high degree, usually exhibiting a 2 to 60 fold volume increase. The osmopolymers can be noncross-linked or cross-linked. The swellable, hydrophilic polymers are in one presently preferred embodiment lightly cross-linked, such cross-links being formed by covalent or ionic bonds or residue crystalline regions after swelling. The osmopolymers can be of plant, animal or synthetic origin. The presently preferred osmopolymers are hydrophilic polymers. Representative of hydrophilic polymers suitable for the present purpose include poly(hydroxy-alkyl methacrylate) having a molecular weight of from 30,000 to 5,000,000; poly(vinylpyrrolidone) having molecular weight of from 10,000 to 360,000; anionic and cationic hydrogels; polyelectrolyte complexes; poly(vinyl alcohol) having a low acetate residual, cross-linked with glyoxal, formaldehyde, or glutaraldehyde and having a degree of polymerization from 200 to 300,000; a mixture of methyl cellulose, cross-linked agar and carboxymethyl cellulose; a mixture of hydroxypropyl methylcellulose and sodium carboxymethylcellulose; a hydroxypropyl methylcellulose and sodium carboxymethyl cellulose; a water insoluble, water swellable copolymer reduced by forming a dispersion of finely divided copolymer of maleic anhydride with styrene, ethylene, propylene, butylene, or isobutylene cross-linked with from 0.001 to about 0.5 moles of saturated cross-linking agent per mole of maleic anhydride in copolymer; water swellable polymers of N-vinyl lactams; polyoxyethylene-polyoxypropylene gel; polyoxybutylene-polyoxyethylene block copolymer gel; carob gum; polyacrylic gel; polyester gel; polyurea gel; polyether gel; polyamide gel; polyamide gel; polypeptide gel; polyamino acid gel; polycellulosic gel; polygum gel; initially dry hydrogels that generally imbibe and absorb water which penetrates the glassy hydrogel and lowers its glass transition temperature.

Other osmopolymers include also polymers that form hydrogels such as Carbopol ® acidic carboxy polymers, a polymer of acrylic acid cross-linked with a polyallyl sucrose, also known as carboxypolymethylene and carboxyvinyl polymer having a molecular weight of 250,000 to 4,000,000; Cyanamer ® polyacrylamides; cross-linked water swellable indene-maleic anhydride polymers; Good-rite ® polyacrylic acid having a molecular weight of 80,000 to 200,000; Polyox ® polyethylene oxide polymers having a molecular weight of 100,000 to 10,000,000 and higher; starch graft copolymers; Aqua-Keeps ® acrylate polymer polysaccharides comprising condensed glucose units such as diester cross-linked polyglucan. Representative polymers that form hydrogels are known to the prior art in U.S. Pat. No. 3,865,108 issued to Hartop; U.S. Pat. No. 4,002,173 issued to Manning; U.S. Pat. No. 4,207,893 issued to Michaels; and in *Handbook of Common Polymers*, by Scott and Roff, published by the Chemical Rubber Company, Cleveland, OH. The amount of osmopolymer 23 is from 10 mg to 250 mg, per second composition 22.

Second composition 22 comprises also a layer forming ingredient 24 identified by dashes, such as a hydroxyalkylcellulose, such as hydroxymethylcellulose, hydroxymethylcellulose, and hydroxypropylcellulose. The concentration of hydroxyalkylcellulose in second composition 23 is 5 mg to 75 mg. Second composition 23 comprises also from 0.1 mg to 3 mg of a lubricant such as stearic acid, magnesium stearate, and zinc stearate.

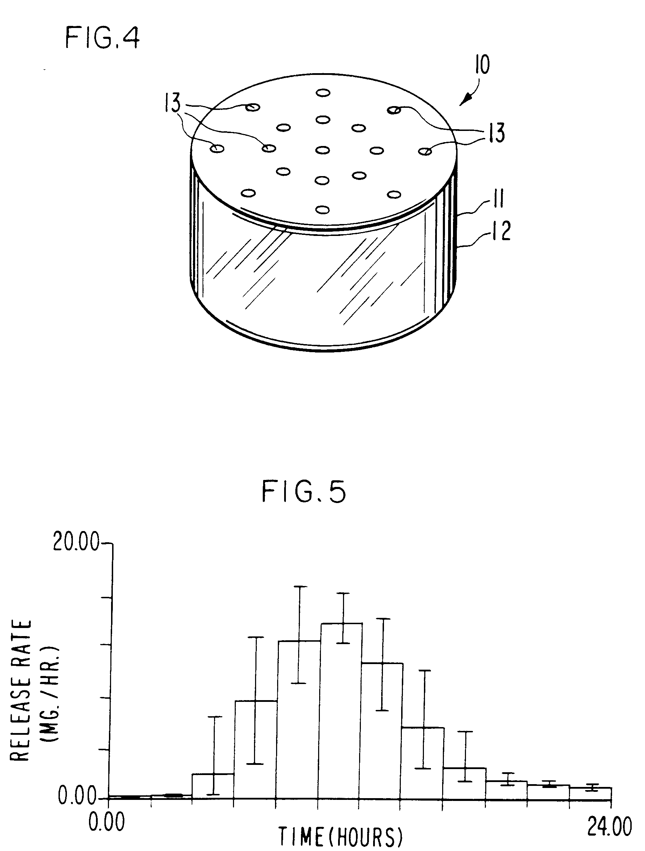

Dosage form 10, as seen in drawing figure 4, comprises at least one passageway 13. The expressions "at least one passageway", includes aperture, orifice, bore, pore, porous element through which the drug can be pumped, diffuse, travel or migrate, hollow fiber, capillary tube, porous overlay, porous insert, and microporous member. The expression also includes a material that erodes or is leached from wall 12 in the fluid environment of use to produce at least one passageway in dosage form 10. Representative material suitable for forming at least one passageway, or a multiplicity of passageways, includes an erodible poly(glycolic) acid or poly(lactic) acid member in the wall; a gelatinous filament; poly(vinyl alcohol); leachable materials such as fluid removable pore forming polysaccharides, salts, or oxides. A passageway or a plurality of passageways can be formed by leaching a material such as sorbitol, sucrose, lactose, or fructose, from the wall. The passageway can have any shape such as round, triangular, square, or elliptical, for assisting in the metered release of drug from dosage form 10. Dosage form 10 can be constructed with one or more passageways in spaced apart relation on one or more than a single surface of a dosage form. Passageways and equipment for forming passages are disclosed in U.S. Pat. Nos. 3,845,770 and 3,916,899 by Theeuwes and Higuchi; in U.S. Pat. No. 4,063,064 by Saunders et al; and in U.S. Pat. No. 4,088,864 by Theeuwes et al. Osmotic passageways of controlled drug releasing dimension, sized, shaped and adapted as a drug releasing pore formed by leaching to provide a drug-releasing pore of controlled osmotic release rate are disclosed in U.S. Pat. No. 4,200,098 by Ayer and Theeuwes; and in U.S. Pat. No. 4,285,987 by Ayer and Theeuwes.

Wall 12 of osmotic dosage form 10 can be formed in one technique using the air suspension procedure. This procedure consists in suspending and tumbling the compressed laminate in a current of air and wall forming composition until a wall is applied to the drug-forming compartment. The air suspension procedure is well-suited for independently forming the wall. The air suspension procedure is described in U.S. Pat. No. 2,799,241; *J*. *A. Pharm. Assoc.*, Vol. 48, pp 451 to 459, 1959; and ibid, Vol. 49, pp 82 to 84, 1960. Osmotic dosage forms can also be coated with a wall-forming composition in a Wurster ® air suspension coater, using methylene dichloride-methanol cosolvent, 80:20, wt:wt, or acetone-water cosolvent, 90:10, wt:wt using 2.5 to 4% solids. The Aeromatic ® air suspension coater using a methylene dichloride-methanol cosolvent, 87:13, wt:wt, also can be used for applying the wall. Other wall forming techniques such as pan coating can be used for providing the dosage form. In the pan coating system, wall forming compositions are deposited by successive spraying of the composition on the trilaminate compartment, accompanying by tumbling in a rotating pan. A pan coater is used to produce thicker walls. A larger volume of solvent, such as methanol can be used in a cosolvent to produce a thinner wall. Finally, the wall coated compartments are dried in a forced air oven at 30°C to 50°C for up to a week to free the dosage form of solvent. Generally, the walls formed by these techniques have a thickness of 2 to 20 mils with a presently preferred thickness of 2 to 15 mils.

Dosage form 10 of the invention is manufactured by standard manufacturing techniques. For example, in one manufacture the beneficial drug and other ingredients comprising the drug-delay composition facing the exit means are blended and pressed into a solid layer. The drug, the delay means and other ingredients can be blended also with a solvent and mixed into a solid or semisolid formed by conventional methods such as ball-milling, calendering, stirring or rollmilling and then pressed into a preselected shape. The first layer possesses dimensions that correspond to the internal dimensions of the area occupied in the dosage form and it also possesses dimensions corresponding to the second push layer for forming a contacting arrangement therewith. The push layer comprising the osmopolymer is placed in contact with the drug layer. The push layer is manufactured using techniques for providing the drug layer. The layering of the drug layer and the push layer can be fabricated by conventional press-layering techniques. Finally, the two-layer compartment forming members are surrounded and coated with an outer wall. A passageway in one manufacture is laser drilled through the wall to contact the delay-drug layer, with the dosage form optically oriented automatically by the laser equipment for forming the passageway on the preselected surface.

In another manufacture, the dosage form is manufactured by the wet granulation technique. In the wet granulation technique, for example, the drug and the ingredients comprising the drug are blended using an organic solvent, such as isopropyl alcohol-methylene dichloride 80:20 v:v (volume:volume) as the granulation fluid. Other granulating fluid such as denatured alcohol 100% can be used for this purpose. The ingredients forming the drug layer are individually passed through a 40 mesh (425 µm) screen and then thoroughly blended in a mixer. Next, other ingredients comprising the drug layer are dissolved in a portion of the granulation fluid, such as the cosolvent described above. Then, the latter prepared wet blend is slowly added to the drug blend with continual mixing in the blender. The granulating fluid is added until a wet blend is produced, which wet mass then is forced through a 20 mesh (850 µm) screen onto oven trays. The blend is dried for 18 to 24 hours at 30°C to 50°C. The dry granules are sized then with a 20 mesh (850 µm) screen. Next, a lubricant is passed through an 80 mesh (180 µm) screen and added to the dry screen granule blend. The granulation is put into milling jars and mixed on a jar mill for 1 to 15 minutes. The delay layer and the push layers are made by the same wet granulation techniques. The compositions are pressed into the individual layers in a Manesty ® two-layer press.

Another manufacturing process that can be used for providing the compartment-forming composition layers comprises blending the powdered ingredients for each layer independently in a fluid bed granulator. After the powdered ingredients are dry blended in the granulator, a granulating fluid, for example poly(vinyl-pyrrolidone) in water, or in denatured alcohol, or in 95:5 ethyl alcohol/water, or in blends of ethanol and water is sprayed onto the powders. Optionally, the ingredients can be dissolved or suspended in the granulating fluid. The coated powders are then dried in a granulator. This process granulates all the ingredients present therein while adding the granulating fluid. After the granules are dried, a lubricant such as stearic acid or magnesium stearate is added to the granulator. The granules for each separate layer are pressed then in the manner described above.

The osmotic device of the invention is manufactured in another embodiment by mixing a drug with composition forming ingredients and pressing the composition into a solid layer possessing dimensions that correspond to the internal dimensions of the compartment. In another embodiment the drug and other delay composition-forming ingredients and a solvent are mixed into a solid, or a semisolid, by conventional methods such as ballmilling, calendering, stirring or rollmilling, and then pressed into a preselected layer forming shape. Next, a layer of a composition comprising an osmopolymer and an optional osmagent are placed in contact with the layer comprising the drug layer. The bilayer can be made by using a conventional two-layer tablet press technique. The wall can be applied by molding, spraying or dipping the pressed shapes into wall-forming materials. Another and presently preferred technique that can be used for applying the wall is the air suspension coating procedure. This procedure consists in suspending and tumbling the two-layered laminate in current of air until the wall forming composition surrounds the laminate. The air suspension procedure is described in U.S. Pat. No. 2,799,241; *J. Am. Pharm. Assoc.*, Vol. 48, pp 451-459 (1979); and, ibid, Vol. 49, pp 82-84 (1960). Other standard manufacturing procedures are described in *Modern Plastics Encyclopedia*, Vol. 46, pp 62-70, (1969); and in *Pharmaceutical Science*, by Remington, 14th Ed., pp 1626-1979, (1970), published by Mack Publishing Co., Easton, PA.

Exemplary solvents suitable for manufacturing the wall, the layers include inert inorganic and organic solvents that do not adversely harm the materials and the final wall. The solvents broadly include members selected for the group consisting of aqueous solvents, alcohols, ketones, esters, ethers, aliphatic hydrocarbons, halogenated solvents, cycloaliphatics, aromatics, heterocyclic solvents and mixtures thereof. Typical solvents include acetone, diacetone, alcohol, methanol, ethanol, isopropyl acetate, n-butyl alcohol, methyl acetate, ethyl acetate, isopropyl acetate, n-butyl acetate, methyl isobutyl ketone, methyl propyl ketone, n-hexane, n-heptane, ethylene glycol, monoethyl ether, ethylene glycol, monoethyl acetate, methylene dichloride, ethylene dichloride, propylene dichloride, carbon tetrachloride, chloroform, nitroethane, nitropropane, tetrachoroethan, ethyl ether, isopropyl ether, cyclohexane, cyclo-octane, benzene, toluene, naphtha, tetrahydrofuran, diglyme, aqueous and nonaqueous mixtures thereof, such as acetone and water, acetone and methanol, acetone and ethyl alcohol, methylene dichloride and methanol, and ethylene dichloride and methanol.

The following examples are merely illustrative of the present invention and they should not be considered as limiting the scope of the invention in any way, as these examples and other equivalents thereof will become apparent to these versed in the art in the light of the present disclosure, the drawing and accompanying claims.

### EXAMPLE 1

A dosage form manufactured as an osmotic device shaped and adapted for oral admittance into the gastrointestinal tract of a human is made as follows: first, a drug composition is prepared by individually screening through a 40 mesh (425 µm) screen verapamil hydrochloride, potassium chloride crystals, mannitol, cross-linked polyvinylpyrrolidone exhibiting a molecular weight greater than 1,000,000, and magnesium stearate. The potassium chloride crystals were dried in an oven for 4 hours at 50°C prior to their granulation to remove moisture. For a 100 g of verapamil drug granulation, 2 g of noncross-linked polyvinylpyrrolidone having a 38,000 molecular weight was dissolved in 30 ml of denature alcohol to prepare a binder solution. The composition forming excipients were mixed next in a standard Hobart ® mixer to produce a consistent wet mixture. The mass of wet granules were passed through a 16 mesh (1.18 mm) screen, spread out on a flat pan and dried in a 50°C forced-air oven ambient humidity for approximately 24 hours to remove the granulation solvent, ethanol. The dried granules were passed through a 16 mesh (1.18 mm) screen. Next, the dried granules were blended with magnesium stearate to lubricate the granules in a V-blender for 2 minutes.

The second composition was prepared by first passing through a 40 mesh (425 µm) screen separating polyethylene oxide comprising a 5,000,000 molecular weight and hydroxypropyl- cellulose comprising a 1,000,000 molecular weight. The two polymers then were granulated in a fluid-bed granulator. Next, approximately 25% of the hydroxypropyl- cellulose was dissolved in sterilized water to prepare a binder solution. The excipients were first mixed in the granulator at room temperature. The granulation was formed at less than 40°C, the melting temperature of hydroxypropylcellulose. The granules formed in the granulator were passed through a 16 mesh (1.18 mm) screen and then magnesium stearate was added to the granules in a blender for 1 minute.

The first composition and the second composition were pressed together into a bilayer with a conventional tablet press. A standard, concave 0.375 inch diameter tool was used to compress the bilayers into an osmotic core. The osmotic layer to drug layer ratio was 1 to 3. The compression force used was approximately 1 ton at a tableting speed of 7.5 rpm.

The bilayer next was coated with a semipermeable wall. The wall-forming composition was prepared as follows: first, a 78/22 methylene chloride/methanol (w:w) solvent was used to dissolve the 35% of cellulose acetate of 39.8% acetyl content, 35% cellulose acetate of 32.0% acetyl content and the hydroxypropylcellulose of molecular weight 60,000 to a 4% weight solid solution. The coating was applied by spraying the wall-forming composition in a 12 inch Freud ® Hi-Coater with an outlet temperature of 34°C, a pump rate of 25 ml/min, a pan rotation speed of 20 rpm and the spray gun to bed distance of 4.75 inches.

A passageway was laser drilled through the wall-coated dosage form on the drug-side surface with a 20 mil diameter. The laser drilled dosage forms were dried in a forced-air oven to remove the methylene chloride and methanol residues retained during coating at ambient humidity and 50°C for 72 hours to yield the final dosage form.

### EXAMPLE 2

An osmotic dosage form is prepared according to Example 1 for administering a therapeutically effective amount of a member selected from the group consisting of nimodipine, nitredipine, nisoldipine, nicardipine, felodipine, diltiazem, lidoflazine, trapemil, isradipine, gallopamil, amlodipine, mioflazine, nilvadipine, and caroverine.

### EXAMPLE 3

An osmotic dosage form is prepared according to Example 2 for administering a therapeutically effective amount of a member selected from the group consisting of amyl nitrate, glyceryl trinitrate, octyl nitrite, sodium nitrite, erythrityl tetranitrate, isosorbide dinitrate, mannitol hexanitrate, pentaerythritol tetranitrate, pentritol, triethanolamine trinitrate, and, trolnitrate phosphate.

### EXAMPLE 4

The procedure of Example 1 is followed in this example, to yield a dosage form comprising: a drug composition comprising 50 wt % verapamil hydrochloride, 25 wt % mannitol, 15 wt % potassium chloride, 9.25 wt % cross-linked polyvinyl pyrrolidone, 0.25 wt % noncross-linked polyvinyl pyrrolidone and 0.50 wt % magnesium stearate, with the drug composition weighing 300 mg; an osmotic push composition weighing 100 mg and comprising 69.50 wt % polyethylene oxide, 29.50 wt % hydroxypropylcellulose, 0.5 wt % ferric oxide, and 0.5 wt % magnesium stearate; a wall comprising 35 wt % cellulose acetate having a 39.8 acetyl content, 35 wt % cellulose acetate having 32.0 acetyl content, and 30 wt % hydroxypropylcellulose; a mean release rate of 10.043 mg/hr between intervals 4 and 8 hours, and an orifice of 20 mil. Accompanying Figure 5 depicts the release rate pattern for the dosage form depicting a 4 hour drug-free interval before the drug delivery period.

### EXAMPLE 5

An osmotic delivery device manufactured in the appearance of an osmotic tablet shaped, sized and adapted for oral admittance into the gastrointestinal tract is made as follows: a first screening 205 g of polyethylene oxide having an approximate molecular weight of 200,000 through a 40 mesh (425 µm) stainless steel screen, then 100 g of diltiazem is pressed through the 40 mesh (425 µm) screen, 25 g of hydroxy- propylcellulose is passed through the screen, 35 g to potassium chloride is passed through the 40 mesh (425 µm) screen and 25 g of sorbitol is passed through the 40 mesh (425 µm) screen. Next, all the screened ingredients are added to the bowl of a laboratory blender and the ingredients dry blended for 15 to 20 minutes to produce a homogenous blend. Then, a granulation fluid is prepared comprising 250 ml of ethanol and 250 ml of isopropyl alcohol, and the granulating fluid added to the blending bowl; first, 50 ml is sprayed into the bowl with constant blending, then 350 ml of the granulation fluid is added slowly to the bowl and the wet mass blended for another 15 to 20 minutes. Then, the wet granules are passed through a 16 mesh (1.18 mm) screen and dried at room temperature for 24 hours. Next, 10 g of calcium stearate is added to the dry granules, and all the ingredients roll-mixed for 20 to 30 minutes on a standard two-roll mill.

Next, a second osmotic composition is prepared as follows: first, 170 g of poly(ethylene oxide) having a molecular weight of 5,000,000 is screened through a 40 mesh (425 µm) screen, then 72.5 g of sodium chloride is passed through the 40 mesh (425 µm) screen, and the ingredients added to a mixing bowl and blended for 10 to 15 minutes. Then, a granulation fluid is prepared by mixing 350 ml of methanol and 150 ml of isopropyl alcohol, and the granulation fluid added to the blending bowl in two steps. First, 50 ml of the granulation fluid is sprayed into the bowl with constant blending; then 350 ml of the granulation fluid is slowly added to the bowl and the wet blend mixed for 15 to 20 minutes to a homogeneous blend. Then, the wet blend is passed through a 16 mesh (1.18 mm) screen, spread on a stainless steel tray and dried at room temperature of 22.5°C for 24 hours. The dried blend is passed through a 16 mesh (1.18 mm) screen, then roll-milled with 5 g of magnesium stearate on a two-roll mill for 20 to 30 minutes.

A number of dry cores are prepared by pressing the two compositions on a Manesty layer press. The drug containing composition is fed into the cavity mold of the press and compressed into a solid layer. Then, the second osmotic composition is fed into the cavity overlaying the compressed layer and pressed into a solid layer to form a two-layered drug core.

The drug cores next are coated with a semipermeable wall forming composition comprising 35 g of cellulose acetate having an acetyl content of 39.8%, 25 g of cellulose acetate having a 32% acetyl content, and 60 g of hydroxypropylcellulose, having a molecular weight of 200,000 in a solvent comprising 1960 ml of methylene chloride and 820 ml of methanol. The drug cores are coated with the semipermeable wall forming composition until the wall surrounds the drug core. A Wurster ® air suspension coater is used to form the semipermeable wall. The' coated cores are then spread on a tray and the solvent evaporated in a circulating air oven at 50°C for 65 hours. After cooling to room temperature, a 0.26 mm diameter passageway is laser drilled through the semipermeable wall connecting the exterior of the osmotic device with the composition containing the drug. The dosage form, after a 4 to 4 1/2 hour drug-free interval deliver drug for 20 hours.

Inasmuch as the foregoing specification comprises preferred embodiments of the invention it is understood that variations and modifications may be made herein, in accordance with the inventive principles disclosed, without departing from the scope of the invention.

## Claims

1. A dosage form (10) for delivering a drug (15) to the gastrointestinal tract of a warm-blooded animal, said dosage form (10) including a compartment which accommodates a first composition including a dosage amount of a drug and a second expandable composition, a semi-permeable wall (12) which surrounds and forms said compartment and exit means (13) in said wall; wherein, in service, fluid from the gastrointestinal tract enters into the compartment by osmosis across the semi-permeable wall thereby forming a solution of said drug (15) and causing or allowing the expandable composition to expand and push against the first composition, thereby to deliver said solution of drug (15) progressively to the gastrointestinal tract via said exit means; characterised in that said first composition further includes delaying means (19) having a greater solubility and a greater osmotic pressure gradient across the semi-permeable wall than the drug (15) such that in service said delaying means (19) is delivered from the dosage form (10) before said drug (15) to provide a substantially drug-free interval.

2. A dosage form as claimed in claim 1 characterised in that the delaying means (19) is selected from one or more of sodium chloride, potassium chloride, magnesium sulfate, magnesium chloride, potassium sulfate, sodium sulfate, potassium acid phosphate, mannitol phosphate, sodium acid phosphate, tartaric acid and citric acid.

3. A dosage form as claimed in claim 1 or claim 2 characterised in that the first drug composition also comprises one or more pharmaceutically acceptable composition forming ingredients selected from a binder, a lubricant, a disintegrant and a colour agent.

4. A dosage form as claimed in claim 3 wherein said disintegrant is selected from poly(vinyl pyrrolidone), lightly cross-linked poly (vinyl pyrrolidone), corn starch, potato starch, bentonite and citrus pulp.

5. A dosage form as claimed in claim 3 or claim 4 wherein in said lubricant is selected from stearic acid, zinc palmitate, magnesium stearate, calcium stearate, zinc stearate, aluminium stearate, magnesium oleate, halogenated vegetable oil, polverised teflon and polverised talc.

6. A dosage form as claimed in any preceding claim wherein the second expandable composition comprises an osmopolymer.

7. A dosage form as claimed in claim 6 wherein the osmopolymer is a cross-linked or non cross-linked swellable, hydrophilic polymer.

8. A dosage form as claimed in claim 6 or claim 7 wherein said osmopolymer is a hydrogel.

9. A dosage form as claimed in any preceding claim wherein the second expandable composition includes a layer forming ingredient and/or a lubricant.

## Patentansprüche

1. Dosierungsform (10) zur Abgabe eines Arzneimittels (15) an den Gastrointestinaltrakt eines Warmblüters, umfassend eine Kammer, die eine erste Zusammensetzung mit einer Arzneimitteldosis und eine zweite ausdehnfähige Zusammensetzung enthält, eine semipermeable Wandung (12), die die Kammer umschließt und bildet, und eine Austrittßvorrichtung (13) in der Wandung, wobei bei der Anwendung Fluid aus dem Gastrointestinaltrakt osmotisch durch die semipermeable Wandung in die Kammer eintritt und dadurch eine Lösung des Arzneimittels (15) herstellt und bewirkt bzw. ermöglicht, daß die ausdehnfähige Zusammensetzung sich ausdehnt und gegen die erste Zusammensetzung drückt, um so die Lösung des Arzneimittels (15) allmählich über die Austrittsvorrichtung an den Gastrointestinaltrakt abzugeben, dadurch **gekennzeichnet**, daß die erste Zusammensetzung ferner ein Verzögerungsmittel (19) enthält, das eine bessere Löslichkeit und einen höheren osmotischen Druckgradienten an der semipermeablen Wandung hat als das Arzneimittel (15) derart, daß bei der Anwendung das Verzögerungsmittel (19) vor dem Arzneimittel (15) aus der Dosierungsform (10) abgegeben wird, um ein im wesentlichen arzneimittelfreies Intervall bereitzustellen.

2. Dosierungsform nach Anspruch 1, dadurch **gekennzeichnet**, daß als Verzögerungsmittel (19) ein oder mehrere Bestandteile der Gruppe ausgewählt sind, die Natriumchlorid, Kaliumchlorid, Magnesiumsulfat, Magnesiumchlorid, Kaliumsulfat, Natriumsulfat, Kaliumdihydrogenphosphat, Mannitphosphat, Natriumdihydrogenphosphat, Weinsäure und Zitronensäure umfaßt.

3. Dosierungsform nach Anspruch 1 oder 2, dadurch **gekennzeichnet**, daß die erste Arzneimittelzusammensetzung ferner eine oder mehrere pharmazeutisch unbedenkliche, eine Zusammensetzung bildende Bestandteile umfaßt, die unter einem Bindemittel, einem Schmiermittel, einem Abbaumittel und einem Farbstoff ausgewählt sind.

4. Dosierungsform nach Anspruch 3, dadurch **gekennzeichnet**, daß das Abbaumittel unter Polyvinylpyrrolidon, leicht vernetztem Polyvinylpyrrolidon, Maisstärke, Kartoffelstärke, Bentonit und Zitronenpülpe ausgewählt ist.

5. Dosierungsform nach Anspruch 3 oder 4, dadurch **gekennzeichnet**, daß das Schmiermittel unter Stearinsäure, Zinkpalmitat, Magnesiumstearat, Calciumstearat, Zinkstearat, Aluminiumstearat, Magnesiumoleat, halogeniertem Pflanzenöl, pulverisiertem Teflon und pulverisiertem Talk ausgewählt ist.

6. Dosierungsform nach einem der vorhergehenden Ansprüche, dadurch **gekennzeichnet**, daß die zweite ausdehnfähige Zusammensetzung ein Osmopolymer enthält.

7. Dosierungsform nach Anspruch 6, dadurch **gekennzeichnet**, daß das Osmopolymer ein vernetztes oder unvernetztes quellfähiges hydrophiles Polymer ist.

8. Dosierungsform nach Anspruch 6 oder Anspruch 7, dadurch **gekennzeichnet**, daß das Osmopolymer ein Hydrogel ist.

9. Dosierungsform nach einem der vorhergehenden Ansprüche, dadurch **gekennzeichnet**, daß die zweite ausdehnfähige Zusammensetzung einen eine Schicht bildenden Bestandteil und/oder ein Schmiermittel enthält.

## Revendications

1. Forme pharmaceutique (10) pour délivrer un médicament (15) dans le tractus gastro-intestinal d'un animal homéotherme, ladite forme pharmaceutique (10) comprenant un compartiment qui contient une première composition comprenant une quantité posologique d'un médicament et une seconde composition expansible, une paroi semi-perméable (12) qui entoure et forme ledit compartiment et un moyen de sortie (13) dans ladite paroi; dans laquelle, lors de son utilisation, un liquide du tractus digestif pénètre dans le compartiment par osmose en traversant la paroi semiperméable, formant ainsi une solution dudit médicament (15) et provoquant ou permettant l'expansion de ladite composition expansible et sa poussée contre la première composition, ce qui permet de délivrer progressivement ladite solution de médicament (15) dans le tractus gastro-intestinal via ledit moyen de sortie; caractérisée en ce que ladite première composition comprend en outre un moyen retardateur (19) dont la solubilité et le gradient de pression osmotique qui s'établit de part et d'autre de la paroi semi-perméable sont supérieurs à ceux du médicament (15), de manière à ce que, lors de l'utilisation de ladite forme pharmaceutique, ledit moyen retardateur (19) soit délivré par la forme pharmaceutique (10) avant ledit médicament (15), ménageant ainsi un intervalle substantiellement sans médicament.

2. Forme pharmaceutique selon la revendication 1 caractérisée en ce que le moyen retardateur (19) est constitué d'un ou de plusieurs membres choisis dans le groupe consistant en chlorure de sodium, chlorure de potassium, sulfate de magnésium, chlorure de magnésium, sulfate de potassium, sulfate de sodium, potassium acide, phosphate de mannitol, phosphate de sodium acide, acide tartrique et acide citrique.

3. Forme pharmaceutique selon la revendication 1 ou la revendication 2 caractérisée en ce que la première composition médicamenteuse comprend en outre un ou plusieurs ingrédients formant la composition acceptables au plan pharmaceutique choisis parmi un liant, un lubrifiant, un agent délitant et un agent colorant.

4. Forme pharmaceutique selon la revendication 3 dans laquelle ledit agent délitant est choisi parmi le poly(vinyl pyrrolidone), le poly(vinyl pyrrolidone) légèrement réticulé, l'amidon de maïs, l'amidon de pomme de terre, la bentonite et la pulpe d'agrumes.

5. Forme pharmaceutique selon la revendication 3 ou la revendication 4 dans laquelle ledit lubrifiant est choisi parmi l'acide stéarique, le palmitate de zinc, le stéarate de magnésium, le stéarate de calcium, le stéarate de zinc, le stéarate d'aluminium, l'oléate de magnésium,, l'huile végétale halogénée, le téflon pulvérisé et le talc pulvérisé.

6. Forme pharmaceutique selon l'une des revendications précédentes dans laquelle la seconde composition expansible comprend un osmopolymère.

7. Forme pharmaceutique selon la revendication 6 dans laquelle l'osmopolymère est un polymère hydrophile, expansible, réticulé ou non réticulé.

8. Forme pharmaceutique selon la revendication 6 ou la revendication 7 dans laquelle ledit osmopolymère est un hydrogel.

9. Forme pharmaceutique selon l'une des revendications précédentes dans laquelle la seconde composition expansible comprend un ingrédient et/ou un lubrifiant formant couche.
